# EUROPEAN PATENT APPLICATION

(11) **EP 4 755 381 A1**
(43) Date of publication of application: **10.06.2026**
(21) Application number: 24849296.9
(22) Date of filing: 02.08.2024
(51) Int. Cl.: A61K 31/4164, A61K 9/08, A61K 47/10, A61K 47/14, A61K 47/20, A61P 17/00

(54) **TOPICAL AGENT FOR ONYCHOMYCOSIS**

(30) Priority: 02.08.2023 JP 2023125977
(71) Applicant: Ishihara Sangyo Kaisha, Ltd., Osaka-shi, Osaka 550-0002 (JP)
(72) Inventor: ISHIBASHI Yutaka, Osaka-shi, Osaka 550-0002 (JP); YAGI Takaharu, Osaka-shi, Osaka 550-0002 (JP)
(74) Representative: Regimbeau
(86) International application number: PCT/JP2024/027707
(87) International publication number: WO 2025/028651

(57) **Abstract**

A topical agent for onychomycosis of the present disclosure contains, as an active ingredient, at least one selected from the group consisting of a compound represented by the following formula (1) and salts thereof, and contains, as a solvent, at least one selected from the group consisting of the following solvents (a) to (g):
wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol, and the solvent (g) is a lower alcohol, a glycol, and a dibasic acid diester.

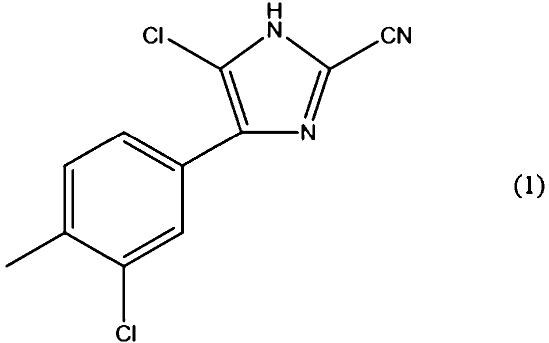

## Description

### Cross-reference to Related Applications

This patent application claims priority to Japanese Patent Application No. 2023 - 125977, filed on August 2, 2023, the entire contents of which are incorporated herein by reference.

### Technical Field

The present disclosure relates to a topical agent for onychomycosis, which is for use in treatment and prevention of exacerbation of onychomycosis.

### Background Art

A compound represented by the following formula (1) (hereinafter, simply referred to as the "compound (1)") is described as a compound No. 6 (Production Example 6) in Patent Literature 1, and is known to be useful as a human antifungal agent. It is also known as a related technology of the compound (1) that a hydrate crystal of the compound (1) possesses properties such as high stability against changes in temperature and humidity, and an ability to reduce frictional electrification, and hence can be advantageously utilized in efficient production of pharmaceutical formulations. However, Patent Literatures 1 and 2 do not specifically describe the use of this compound for treating onychomycosis, and naturally, neither disclose nor suggest any formulation prescription for a topical agent for onychomycosis.

Onychomycosis is a disease caused by trichophyton infection of the nail, and as a treatment for this disease, oral or topical medications are used.

Oral medications are currently mainly used, but these medications have often been criticized for several drawbacks, including the necessity for patients to endure long-term continuous administration, the potential for interactions with other medications, and the risk of side effects such as liver dysfunction.

Therefore, there has been a strong desire for development of effective topical medications. A main issue with topical medications is difficulty of delivering the medication, in an amount sufficient for exhibiting the treatment effect, to the affected site, and studies are mainly focused on, for example, improving penetration (permeation) into the nail, suppressing crystal precipitation during storage and application, and stabilizing the active ingredient.

As one such investigation, topical pharmaceutical compositions containing luliconazole or lanoconazole as the active ingredient are known (see Patent Literatures 3 to 5).

According to Patent Literature 3, the phenomenon unique to luliconazole, in which fine crystals of luliconazole instantaneously precipitate upon application to inhibit permeation and absorption into biological tissue, can be prevented by incorporating an α-hydroxycarboxylic acid or a salt thereof. A dibasic acid diester is listed as one of optional ingredients and is described as an ingredient that promotes penetration into the nail.

Patent Literature 4 describes that N-methyl-2-pyrrolidone exhibits the effect of suppressing the precipitation of crystals or insoluble matter on a surface of the application site, which appears 20 to 40 seconds after the administration and is considered to be due to an interaction with the surface structure of the skin or nail or an interaction with substances present on the surface. Also in Patent Literature 4, a dibasic acid diester is listed as an optional ingredient and is described as an ingredient that promotes penetration into the nail.

Patent Literature 5 describes that desired properties, such as suppression of active ingredient isomerization, transparency at 20°C after production, and prevention of precipitation during low-temperature storage, are achieved by containing at least one selected from the group consisting of a prescribed higher alcohol and a dibasic acid diester, and at least one selected from the group consisting of a polyoxyethylene alkyl ether and a polyoxyethylene alkenyl ether. Patent Literature 5 describes that one of the reasons for incorporating the dibasic acid diester is for suppression of crystal precipitation in a low-temperature region, and also that when it is used with a prescribed higher alcohol in a specific ratio, an inhibitory effect is also exhibited on crystal precipitation caused by impact, such as crystal precipitation upon application to the nail.

Furthermore, a topical antifungal agent containing efinaconazole as the active ingredient is also known (see Patent Literature 6). Patent Literature 6 describes that effective transungual penetration is achieved by containing efinaconazole, a monohydric alcohol, a glycol, and a prescribed excipient. A large number of examples of the excipient are listed, including dimethyl sulfoxide and a dibasic acid diester, and it is understood, in view of the overall object of the invention, that the purpose of incorporating is promotion of penetration into the nail.

As described above, formulation studies for topical medications have been conducted on a plurality of active ingredients, but absolutely no investigation has been conducted on a topical agent for onychomycosis that uses, as the active ingredient, at least one selected from the group consisting of the compound (1) and salts thereof.

Although the aforementioned literatures describe the incorporation of dimethyl sulfoxide and a dibasic acid diester, it is general knowledge in formulation studies that the optimal formulation will differ due to differences in the active ingredient and others. Therefore, it cannot be said that the aforementioned literatures, which describe the active ingredients different from that of the present disclosure, suggest a formulation for a topical agent for onychomycosis in which at least one selected from the group consisting of the compound (1) and salts thereof is used as the active ingredient, and in which a specific solvent of the present disclosure defined below is incorporated.

In relation to this point, paragraph [0029] of Patent Literature 3 describes an evaluation of the degree of crystal precipitation after coating, onto a slide glass, compositions of Example 5 (a lotion containing luliconazole, diisopropyl adipate, lactic acid, and ethanol) and Comparative Example 6, in which the lactic acid in the formulation of Example 5 is replaced with ethanol (namely, a lotion containing luliconazole, diisopropyl adipate, and ethanol). It is described that, in the latter composition, crystal precipitation was not suppressed (also see Figures 4 and 5 of Patent Literature 3).

### Citation List

### Patent Literature

Patent Literature 1: WO2021/246455
Patent Literature 2: WO2023/106320
Patent Literature 3: WO2007/102241
Patent Literature 4: WO2007/102243
Patent Literature 5: WO2010/117091
Patent Literature 6: WO2020/231800

### Summary of Invention

### Technical Problem

An object of the present disclosure is to provide a topical agent for onychomycosis that contains, as an active ingredient, at least one selected from the group consisting of a compound (1) and salts thereof, and can exhibit sufficient efficacy of the active ingredient on onychomycosis by suppressing crystal precipitation in a formulation and crystal precipitation after application, and improving nail penetration.

### Solution to Problem

The present inventors have conducted extensive studies to solve the above-described problems and have found that when a specific solvent is used, crystal precipitation in the formulation and crystal precipitation after the application are suppressed, nail penetration is improved, and sufficient efficacy can be exhibited on onychomycosis, and thus, the present invention has been accomplished.

Specifically, the present disclosure provides a topical agent for onychomycosis containing, as an active ingredient, at least one selected from the group consisting of a compound represented by the following formula (1) and salts thereof, and further containing, as a solvent, at least one selected from the group consisting of the following solvents (a) to (g):

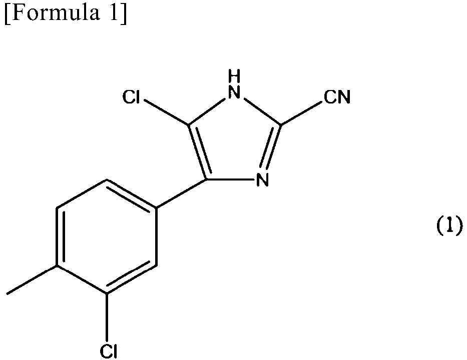

The solvent (a): a dibasic acid diester;
the solvent (b): a lower alcohol and a dibasic acid diester;
the solvent (c): a lower alcohol and dimethyl sulfoxide;
the solvent (d): dimethyl sulfoxide and a dibasic acid diester;
the solvent (e): a lower alcohol, dimethyl sulfoxide, and a dibasic acid diester;
the solvent (f): a lower alcohol, and a glycol; and
the solvent (g): a lower alcohol, a glycol, and a dibasic acid diester.

### Advantageous Effects of Invention

According to the present disclosure, at least one compound selected from the group consisting of the compound (1) and salts thereof, which is the active ingredient, can be prevented from crystal precipitation in the formulation and crystal precipitation after the application, can achieve improved nail penetration, and can sufficiently exhibit the efficacy on onychomycosis.

### Brief Description of Drawing

[Figure 1] Figure 1 is a photograph showing the state of a topical agent after standing for 24 hours, regarding an evaluation test "Suppression of Crystal Precipitation in Formulation" performed in Example 1.
[Figure 2] Figure 2 is a photograph showing the state of a topical agent after standing for 24 hours, regarding an evaluation test "Suppression of Crystal Precipitation in Formulation" performed in Comparative Example 3.
[Figure 3] Figure 3 is a photograph showing the state of a topical agent on a glass plate after a prescribed period following the application of the topical agent, regarding an evaluation test "Suppression of Crystal Precipitation after Application" performed in Example 22.
[Figure 4] Figure 4 is a photograph showing the state of a topical agent on a glass plate after a prescribed period following the application of the topical agent, regarding an evaluation test "Suppression of Crystal Precipitation after Application" performed in Comparative Example 1.
[Figure 5] Figure 5 is a photograph showing the state of a topical agent on a glass plate after a prescribed period following the application of the topical agent, regarding an evaluation test "Suppression of Crystal Precipitation after Application" performed in Comparative Example 2.
[Figure 6] Figure 6 is photographs showing the condition around the nail when conducting a subungual efficacy test of topical agents of Examples 68 and 69, and placebos and a control thereof.
[Figure 7] Figure 7 is photographs showing fluorescence staining results obtained when conducting a subungual fungicidal activity test of the topical agent of Example 68, a placebo thereof, and two commercially available drugs.
[Figure 8] Figure 8 is a graph showing the change in the amount of hydrolysate generated when the concentration of lactic acid is varied in a formulation of Example 49 or Example 59.
[Figure 9] Figure 9 is a graph showing a difference in the amount of hydrolysate generated in Reference Examples 1 to 11 different both or either of the type and the amount of acid added.

### Description of Embodiments

A topical agent for onychomycosis of the present disclosure (hereinafter also simply referred to as the "topical agent of the present disclosure") will now be described in detail. However, the scope of the present disclosure is not limited by these descriptions, and modifications may be implemented as appropriate, even in cases other than examples described below, without departing from the spirit of the present disclosure.

A topical agent of the present disclosure contains, as an active ingredient, at least one compound selected from the group consisting of a compound (1) and salts thereof.

The compound (1) is a known compound as described above, and is represented by a chemical name, 5-chloro-4-(3-chloro-4-methylphenyl)-1H-imidazole-2-carbonitrile.

The salts of the compound (1) are not especially limited as long as they are pharmaceutically acceptable salts, and examples include inorganic acid salts such as hydrochloride, hydrobromide, sulfate, nitrate, and phosphate; and organic acid salts such as acetate, propionate, tartrate, fumarate, maleate, malate, citrate, methane sulfonate, benzenesulfonate, and p-toluenesulfonate. Examples of a basic salt include alkali metal salts such as sodium salt and potassium salt, and alkaline earth metal salts such as calcium salt and magnesium salt.

The at least one compound selected from the group consisting of the compound (1) and salts thereof may exist as the following tautomers. However, the at least one compound selected from the group consisting of the compound (1) and salts thereof in the present disclosure encompasses both a case where it exists as any one of these tautomers and a case where the tautomers coexist (including a case where they coexist in an equilibrium state).

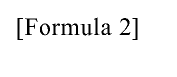

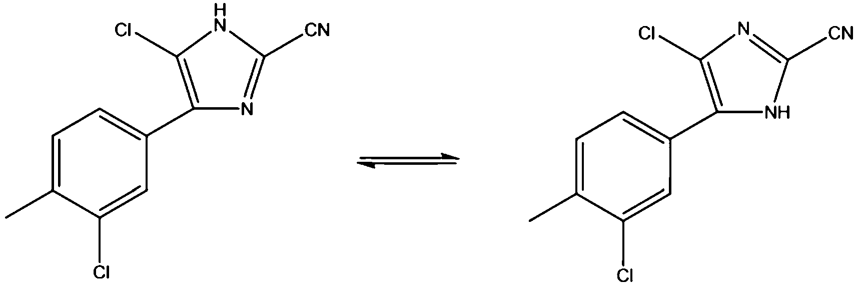

When the compound (1) or salt thereof is left in the atmosphere or subjected to recrystallization, it may absorb moisture to become associated with adsorbed water or become a hydrate. In the present disclosure, the compound (1) or salt thereof also encompasses such various hydrates, solvates, and crystalline polymorphic compounds. Preferable examples include a hydrate and a crystalline polymorphic compound disclosed in WO2023/106320.

The content of the at least one compound selected from the group consisting of the compound (1) and salts thereof in the entire amount of the topical agent of the present disclosure is not especially limited, and for example, is preferably 0.1 to 20% by mass, more preferably 0.5 to 10% by mass, and particularly preferably 1 to 5% by mass.

The topical agent of the present disclosure contains, as a solvent, at least one solvent selected from the group consisting of the following solvents (a) to (g) (hereinafter also simply referred to as the "specific solvent of the present disclosure"):
The solvent (a): a dibasic acid diester;
the solvent (b): a lower alcohol and a dibasic acid diester;
the solvent (c): a lower alcohol and dimethyl sulfoxide;
the solvent (d): dimethyl sulfoxide and a dibasic acid diester;
the solvent (e): a lower alcohol, dimethyl sulfoxide, and a dibasic acid diester;
the solvent (f): a lower alcohol, and a glycol; and
the solvent (g): a lower alcohol, a glycol, and a dibasic acid diester.

The dibasic acid diester used in the specific solvent of the present invention is a compound formed by diesterification of a dibasic acid and an alcohol.

The dibasic acid is not especially limited, and an aliphatic dibasic acid or an aromatic dibasic acid is preferably used.

Examples of the aliphatic dibasic acid include saturated aliphatic dibasic acids such as oxalic acid, malonic acid, succinic acid, glutaric acid, adipic acid, pimelic acid, suberic acid, azelaic acid, and sebacic acid; and unsaturated aliphatic dibasic acids such as maleic acid and fumaric acid. An aliphatic dibasic acid having 2 to 10 carbon atoms is preferred, an aliphatic dibasic acid having 2 to 6 carbon atoms is more preferred, and at least one selected from the group consisting of adipic acid, fumaric acid, and maleic acid is particularly preferred.

Examples of the aromatic dibasic acid include phthalic acid, isophthalic acid, and terephthalic acid.

The alcohol that is diesterified with the dibasic acid is not especially limited, and preferable examples include alcohols having 1 to 4 carbon atoms, such as methanol, ethanol, propanol, isopropyl alcohol, butanol, isobutyl alcohol, and tert-butyl alcohol. At least one selected from the group consisting of ethanol, isopropyl alcohol, butanol, and isobutyl alcohol is more preferred.

The dibasic acid diester is not especially limited, and preferable examples include diisopropyl adipate, diisobutyl adipate, dibutyl fumarate, and diethyl maleate. Diisopropyl adipate is particularly preferred.

The lower alcohol used in the specific solvent of the present disclosure is not especially limited, and preferable examples include alcohols having 1 to 4 carbon atoms such as methanol, ethanol, propanol, isopropyl alcohol, butanol, isobutyl alcohol, and tert-butyl alcohol, and at least one selected from the group consisting of ethanol and isopropyl alcohol is particularly preferred.

The glycol used in the specific solvent of the present disclosure is not especially limited, and preferable examples include ethylene glycol, propylene glycol, 1,3-propanediol, 1,4-butanediol, and dehydration condensates of these. Preferable examples of the dehydration condensates include diethylene glycol, triethylene glycol, polyethylene glycol, dipropylene glycol, tripropylene glycol, and polypropylene glycol. Regarding the molecular weight, the number average molecular weight of the glycol in the form of a dehydration condensate is, for example, preferably 200 to 600, and more preferably 200 to 400. Propylene glycol and polyethylene glycol are particularly preferred.

The content of the specific solvent of the present disclosure relative to the total amount of the topical agent of the present disclosure is not especially limited, and is preferably 60 to 99.9% by mass, more preferably 70 to 99.9% by mass, and particularly preferably 80 to 99.9% by mass relative to the total amount of the topical agent of the present disclosure.

In the solvent (b), the blending ratio between the lower alcohol and the dibasic acid diester, i.e., lower alcohol:dibasic acid diester, is preferably 99.9:0.1 to 10:90, and more preferably 75:25 to 50:50 on a mass basis.

In the solvent (c), the blending ratio between the lower alcohol and the dimethyl sulfoxide, i.e., lower alcohol:dimethyl sulfoxide, is preferably 99.9:0.1 to 10:90, and more preferably 75:25 to 50:50 on a mass basis.

In the solvent (d), the blending ratio between the dimethyl sulfoxide and the dibasic acid diester, i.e., dimethyl sulfoxide:dibasic acid diester, is preferably 99.9:0.1 to 10:90, and more preferably 75:25 to 50:50 on a mass basis.

In the solvent (e), the blending ratio between the lower alcohol, the dimethyl sulfoxide, and the dibasic acid diester is as follows: when the total of the three components is defined as 100 mass%, preferably the lower alcohol accounts for 0.1 to 99.8 mass%, the dimethyl sulfoxide for 0.1 to 99.8 mass%, and the dibasic acid diester for 0.1 to 99.8 mass%; and preferably the lower alcohol accounts for 10 to 75 mass%, the dimethyl sulfoxide for 5 to 75 mass%, and the dibasic acid diester for 10 to 75 mass%.

In the solvent (f), the blending ratio between the lower alcohol and the glycol, i.e., lower alcohol:glycol, is preferably 99.9:0.1 to 10:90, and more preferably 75:25 to 50:50 on a mass basis.

In the solvent (g), the blending ratio between the lower alcohol, the glycol, and the dibasic acid diester is as follows: when the total of the three components is defined as 100 mass%, preferably the lower alcohol accounts for 0.1 to 99.8 mass%, the glycol for 0.1 to 99.8 mass%, and the dibasic acid diester for 0.1 to 99.8 mass%; and preferably the lower alcohol accounts for 55 to 90 mass%, the glycol for 5 to 35 mass%, and the dibasic acid diester for 5 to 35 mass%.

In the topical agent of the present disclosure, the blending ratio between the at least one compound selected from the group consisting of the compound (1) and salts thereof, and the specific solvent of the present disclosure is not especially limited, and is preferably 0.1:99.9 to 40:60, more preferably 0.1:99.9 to 20:80, and particularly preferably 1:99 to 10:90 on a mass basis.

The topical agent of the present disclosure may also contain any solvent used in pharmaceutical compositions, in addition to the specific solvent of the present disclosure.

Examples of such another solvent include triglycerides such as olive oil, fatty acids such as stearic acid and oleic acid, and polyhydric alcohols other than glycols (e.g., glycerin).

N-methyl-2-pyrrolidone is suitable as a solvent for at least one compound selected from the group consisting of the compound (1) and salts thereof; however, it does not contribute to the suppression of crystal precipitation and may even inhibit the effect of suppressing crystal precipitation of the present disclosure. Therefore, it is preferable not to use it, or to use it only in a small amount, if any. In the latter case, the amount used is preferably 10% by mass or less, more preferably 5% by mass or less, and particularly preferably 1% by mass or less relative to the total amount of the topical agent of the present disclosure.

The topical agent of the present disclosure may also contain any ingredient, in addition to the above-mentioned ingredients, as an optional ingredient.

As such an optional ingredient, for example, general pharmaceutical additives for topical agents can be used, including an extender, a diluent, a pH adjusting agent, a dispersing agent, an emulsifier, a preservative, a stabilizing agent, an antioxidant, a coloring agent, an ultraviolet absorber, a humectant, and a thickening agent.

Particularly preferable examples of the optional ingredient are as follows.

A specific example of the preferable optional ingredient is polyvinylpyrrolidone. Polyvinylpyrrolidone has the effect of suppressing crystal precipitation after application. This effect is presumed to be due to polyvinylpyrrolidone causing the at least one compound selected from the group consisting of the compound (1) and salts thereof to become amorphous.

The molecular weight of the polyvinylpyrrolidone is not especially limited, and for example, one having a weight average molecular weight of 10,000 to 360,000 can be used.

The amount of the polyvinylpyrrolidone used is not especially limited, and is preferably 0.1 to 10% by mass, and more preferably 0.5 to 5% by mass relative to the total amount of the topical agent of the present disclosure.

Examples of an ingredient (crystallization inhibitor) that is expected to have a similar effect to polyvinylpyrrolidone (suppression of crystallization through amorphization) include polymeric compounds such as an ethylene-maleic anhydride copolymer and polyalkylene glycols (e.g., polyethylene glycol, polyoxyethylene-polyoxypropylene glycol), and these compounds may be used as the optional ingredient in the topical agent of the present disclosure.

An acid is preferably incorporated as the pH adjusting agent. By incorporating an acid as the pH adjusting agent, the stability of the at least one compound selected from the group consisting of the compound (1) and salts thereof is improved, which leads to an improvement in the penetration ability into the living body. Preferable specific examples of the pH adjusting agent include lactic acid, acetic acid, citric acid (including monohydrate), ascorbic acid, and phosphoric acid. Lactic acid and citric acid are more preferred, and lactic acid is particularly preferred.

The amount of the acid used as the pH adjusting agent is not especially limited, and is preferably 0.1 to 10% by mass, and more preferably 0.5 to 5% by mass relative to the total amount of the topical agent of the present disclosure. Particularly when lactic acid is incorporated, the amount, in terms of the upper limit, is preferably 8% by mass or less, more preferably 4% by mass or less, and particularly preferably the amount is 0.5 to 2% by mass.

As the preservative, it is preferable to incorporate, for example, benzyl alcohol, benzoic acid and salts thereof (such as a sodium salt), sebacic acid, and sorbic acid, and benzyl alcohol is particularly preferred.

The amount of the preservative used is not especially limited, and is preferably 0.1 to 10% by mass, and more preferably 0.1 to 5% by mass relative to the total amount of the topical agent of the present disclosure.

The topical agent of the present disclosure can be obtained by formulating the above-mentioned essential ingredients and, if any, the optional ingredients in combination by a conventional method.

The dosage form of the topical agent can be a general form common for topical agents, such as a lotion, a cream, an emulsion, a suspension, a gel, or a patch, and a lotion is particularly suitable.

The topical agent of the present disclosure can be used for treatment or prevention of exacerbation of onychomycosis, and the agent can be applied locally to the nail that is the affected area of a target animal species (e.g., a human) by application, rubbing, patching, or the like, depending on the specific dosage form thereof.

The effective amount of the at least one compound selected from the group consisting of the compound (1) and salts thereof, and the frequency of administration of the topical agent of the present disclosure are not especially limited, and are difficult to be unconditionally defined because these vary depending on the animal species, sex, age, body weight, condition, and other factors of the subject of administration. For example, when the agent is locally administered to the affected area of a human, the dosage, in terms of the amount of the at least one compound selected from the group consisting of the compound (1) and salts thereof, is usually 500 to 3,000 µg/day per nail as a daily dose, and a preferred dosage per week is 3.5 to 15 mg/week per nail for an adult weighing 60 kg.

Another aspect of the present disclosure provides a method for treating onychomycosis in a subject, the method including locally applying an effective amount of a topical agent for onychomycosis to an affected nail of the subject, wherein the topical agent for onychomycosis contains, as an active ingredient, at least one selected from the group consisting of the compound represented by the above-described formula (1) and salts thereof, and further contains, as a solvent, at least one selected from the group consisting of the above-described solvents (a) to (g). Hereinafter, this method is also referred to simply as the "treatment method".

Another aspect of the present disclosure provides a method for preventing exacerbation of onychomycosis in a subject, including locally applying an effective amount of a topical agent for onychomycosis to an affected nail of the subject, wherein the topical agent for onychomycosis contains, as an active ingredient, at least one selected from the group consisting of the compound represented by the above-described formula (1) and salts thereof, and further contains, as a solvent, at least one selected from the group consisting of the above-described solvents (a) to (g). Hereinafter, this method is also referred to simply as the "prevention method".

In the treatment and prevention methods of the present disclosure, "treatment of onychomycosis" encompasses stopping, alleviating, or delaying the progression or exacerbation of onychomycosis by medical or non-medical procedure. Furthermore, "prevention of exacerbation of onychomycosis" encompasses preparing in advance for the anticipated exacerbation of onychomycosis, and preventing the occurrence or recurrence by medical or non-medical procedure.

In the treatment and prevention methods of the present disclosure, the "effective amount of the topical agent for onychomycosis" is difficult to be unconditionally defined because it varies depending on the animal species, sex, age, body weight, condition, and other factors of the subject of administration. For example, when the agent is locally administered to the affected area of a human, the dosage, in terms of the amount of the at least one compound selected from the group consisting of the compound (1) and salts thereof, is usually 500 to 3,000 µg/day per nail per day, and a preferred dosage is 3.5 to 15 mg/week per person per nail for an adult weighing 60 kg.

In the treatment and prevention methods of the present disclosure, the topical agent for onychomycosis can be applied locally to the nail that is the affected area of the target animal species (e.g., a human) by application, rubbing, patching, or the like depending on the specific dosage form thereof.

In the treatment and prevention methods of the present disclosure, the topical agent for onychomycosis contains, as a solvent, at least one selected from, for example, the group consisting of the solvents (a), (b), (d), and (e) described above. Alternatively, the agent may contain, as a solvent, at least one selected from the group consisting of the solvents (c), (d), and (e). Further alternatively, the agent may contain, as a solvent, at least one selected from the group consisting of the solvents (d) and (e).

In the treatment and prevention methods of the present disclosure, the dibasic acid diester is preferably a diester of a dibasic acid and an alcohol having 1 to 4 carbon atoms.

In the treatment and prevention methods of the present disclosure, the topical agent for onychomycosis preferably further contains polyvinylpyrrolidone.

In the treatment and prevention methods of the present disclosure, the topical agent for onychomycosis preferably further contains an acid as a pH adjusting agent, and the pH adjusting agent is more preferably lactic acid or citric acid, and further preferably lactic acid.

In the topical agent for onychomycosis, the content of the at least one selected from the group consisting of the compound (1) and salts thereof is preferably 0.1 to 20% by mass, and the content of the at least one selected from the group consisting of the solvents (a) to (g) is preferably 60 to 99.9% by mass.

A still another aspect of the present disclosure provides use, for producing a topical agent for onychomycosis or a medication for treating or preventing exacerbation of onychomycosis, of a combination of at least one compound selected from the group consisting of the compound represented by the formula (1) and salts thereof, and at least one solvent selected from the group consisting of the solvents (a) to (g). Hereinafter, this use is also referred to as the "use of the compound combination" or "combination use of the compounds".

A still another aspect of the present disclosure provides use, for producing a topical agent for onychomycosis or a medication for treating or preventing exacerbation of onychomycosis, of a composition containing, as an active ingredient, at least one selected from the group consisting of the compound represented by the above-described formula (1) and salts thereof, and further containing, as a solvent, at least one selected from the group consisting of the above-described solvents (a) to (g). Hereinafter, this use is also referred to as the "use of the composition".

In the use of the compound combination and the use of the composition of the present disclosure, the "treatment of onychomycosis" and "prevention of exacerbation of onychomycosis" are as defined above.

In the use of the compound combination and the use of the composition of the present disclosure, the topical agent for onychomycosis or the medication contains, as a solvent, at least one selected from, for example, the group consisting of the solvents (a), (b), (d), and (e) described above. Alternatively, it may contain, as a solvent, at least one selected from the group consisting of the solvents (c), (d), and (e). Further alternatively, it may contain, as a solvent, at least one selected from the group consisting of the solvents (d) and (e).

In the use of the compound combination and the use of the composition of the present disclosure, the dibasic acid diester is preferably a diester of a dibasic acid and an alcohol having 1 to 4 carbon atoms.

In the use of the compound combination and the use of the composition of the present disclosure, the topical agent for onychomycosis and the medication preferably further contain polyvinylpyrrolidone.

In the use of the compound combination and the use of the composition of the present disclosure, the topical agent for onychomycosis and the medication preferably further contain an acid as a pH adjusting agent, and the pH adjusting agent is more preferably lactic acid or citric acid, and further preferably lactic acid.

In the topical agent for onychomycosis and the medication, the content of the at least one selected from the group consisting of the compound (1) and salts thereof is preferably 0.1 to 20% by mass, and the content of the at least one selected from the group consisting of the solvents (a) to (g) is preferably 60 to 99.9% by mass.

A still another aspect of the present invention provides a composition, for use in treatment or prevention of exacerbation of onychomycosis, containing, as an active ingredient, at least one selected from the group consisting of the compound represented by the above-described formula (1) and salts thereof, and further containing, as a solvent, at least one selected from the group consisting of the above-described solvents (a) to (g). Hereinafter, this is also referred to simply as the "composition".

In the composition of the present disclosure, the "treatment of onychomycosis" and "prevention of exacerbation of onychomycosis" are as defined above.

The composition of the present disclosure is applied locally to the nail that is the affected area of a target animal species (e.g., a human) by application, rubbing, patching, or the like depending on the specific dosage form thereof.

The composition of the present disclosure contains, as a solvent, at least one selected from, for example, the group consisting of the solvents (a), (b), (d), and (e) described above. Alternatively, the composition may contain, as a solvent, at least one selected from the group consisting of the solvents (c), (d), and (e). Further alternatively, the composition may contain, as a solvent, at least one selected from the group consisting of the solvents (d) and (e).

In the composition of the present disclosure, the dibasic acid diester is preferably a diester of a dibasic acid and an alcohol having 1 to 4 carbon atoms.

The composition of the present disclosure preferably further contains polyvinylpyrrolidone.

The composition of the present disclosure preferably further contains an acid as a pH adjusting agent, and the pH adjusting agent is more preferably lactic acid or citric acid, and further preferably lactic acid.

In the composition of the present disclosure, the content of the at least one selected from the group consisting of the compound (1) and salts thereof is preferably 0.1 to 20% by mass, and the content of the at least one selected from the group consisting of the solvents (a) to (g) is preferably 60 to 99.9% by mass.

One aspect of the present disclosure provides the following:
[1] A topical agent for onychomycosis containing, as an active ingredient, at least one selected from the group consisting of a compound represented by the following formula (1) and salts thereof, and further containing, as a solvent, at least one selected from the group consisting of the following solvents (a) to (g): 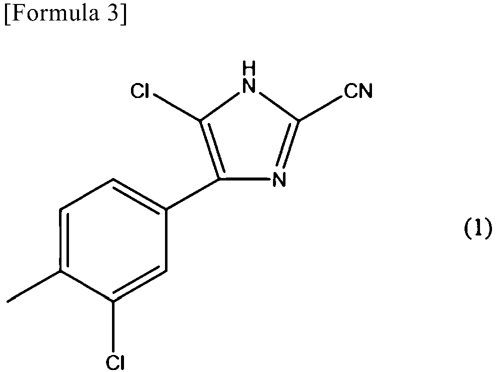 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
[2] The topical agent for onychomycosis according to [1], containing, as a solvent, at least one selected from the group consisting of the solvents (a), (b), (d), and (e).
[3] The topical agent for onychomycosis according to [1] or [2], containing, as a solvent, at least one selected from the group consisting of the solvents (c), (d), and (e).
[4] The topical agent for onychomycosis according to any one of [1] to [3], containing, as a solvent, at least one selected from the group consisting of the solvents (d) and (e).
[5] The topical agent for onychomycosis according to [1] to [4], wherein the dibasic acid diester is a diester of a dibasic acid and an alcohol having 1 to 4 carbon atoms.
[6] The topical agent for onychomycosis according to any one of [1] to [5], further containing polyvinylpyrrolidone.
[7] The topical agent for onychomycosis according to any one of [1] to [6], further containing an acid as a pH adjusting agent.
[8] The topical agent for onychomycosis according to [7], wherein the pH adjusting agent is lactic acid or citric acid.
[9] The topical agent for onychomycosis according to any one of [1] to [8], wherein a content of the at least one selected from the group consisting of the compound (1) and salts thereof is 0.1 to 20% by mass, and a content of the at least one selected from the group consisting of the solvents (a) to (g) is 60 to 99.9% by mass.
[10] Use, for producing a topical agent for onychomycosis or a medication for treating or preventing exacerbation of onychomycosis, of a combination of at least one compound selected from the group consisting of a compound represented by the following formula (1) and salts thereof, and at least one solvent selected from the group consisting of the following solvents (a) to (g): 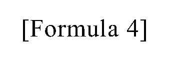 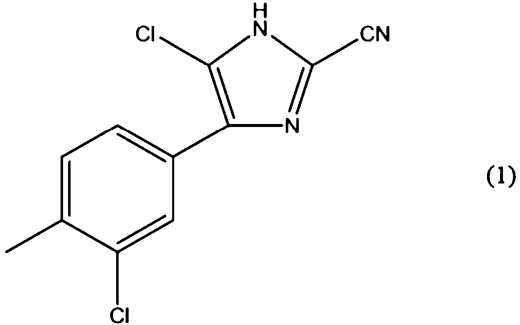 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
[11] Use, for producing a topical agent for onychomycosis or a medication for treating or preventing exacerbation of onychomycosis, of a composition containing, as an active ingredient, at least one selected from the group consisting of a compound represented by the following formula (1) and salts thereof, and further containing, as a solvent, at least one selected from the group consisting of the following solvents (a) to (g): 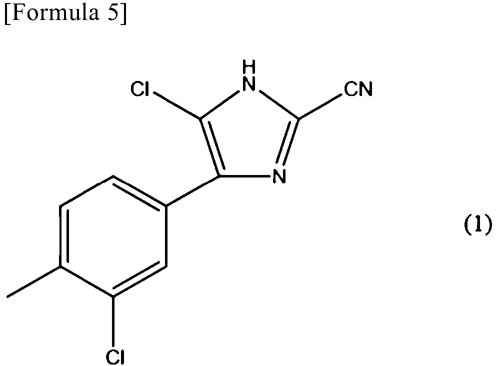 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
[12] A composition, for use in treatment or prevention of exacerbation of onychomycosis, containing, as an active ingredient, at least one selected from the group consisting of a compound represented by the following formula (1) and salts thereof, and further containing, as a solvent, at least one selected from the group consisting of the following solvents (a) to (g): 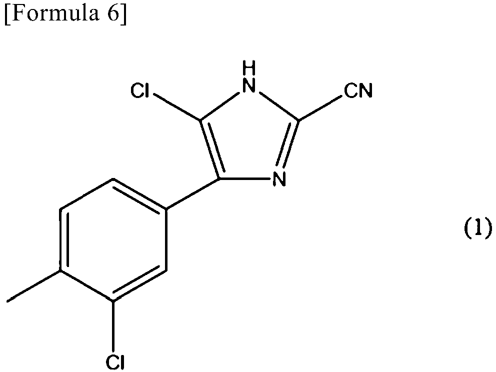 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
[13] A method for treating onychomycosis in a subject, including locally applying an effective amount of a topical agent for onychomycosis to an affected nail of the subject, wherein the topical agent for onychomycosis contains, as an active ingredient, at least one selected from the group consisting of a compound represented by the following formula (1) and salts thereof, and further contains, as a solvent, at least one selected from the group consisting of the following solvents (a) to (g): 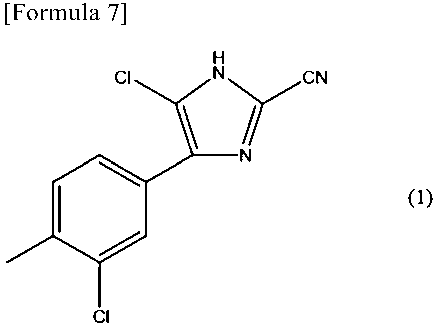 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
[16] A method for preventing exacerbation of onychomycosis in a subject, including locally applying an effective amount of a topical agent for onychomycosis to an affected nail of the subject, wherein the topical agent for onychomycosis contains, as an active ingredient, at least one selected from the group consisting of a compound represented by the following formula (1) and salts thereof, and further contains, as a solvent, at least one selected from the group consisting of the following solvents (a) to (g): 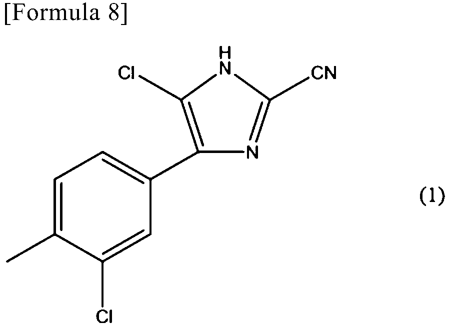 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.

Another aspect of the present disclosure provides the following:
[T1] Use, for producing a topical agent for onychomycosis or a medication for treating or preventing exacerbation of onychomycosis, of a combination of at least one compound selected from the group consisting of a compound represented by the following formula (1) and salts thereof, and at least one solvent selected from the group consisting of the following solvents (a) to (g): 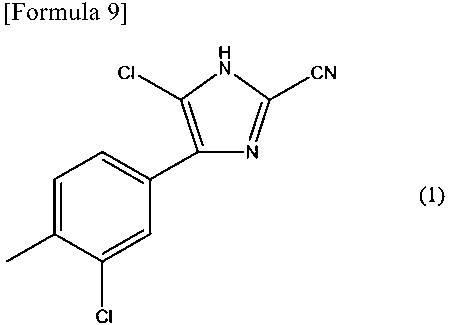 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
[T2] Use, for producing a topical agent for onychomycosis or a medication for treating or preventing exacerbation of onychomycosis, of a composition containing, as an active ingredient, at least one selected from the group consisting of a compound represented by the following formula (1) and salts thereof, and further containing, as a solvent, at least one selected from the group consisting of the following solvents (a) to (g): 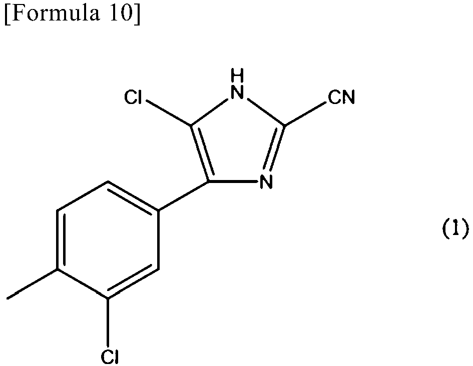 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
[T3] The use according to [T1] or [T2], wherein the topical agent for onychomycosis or the medication contains, as a solvent, at least one selected from the group consisting of the solvents (a), (b), (d), and (e).
[T4] The use according to any one of [T1] to [T3], wherein the topical agent for onychomycosis or the medication contains, as a solvent, at least one selected from the group consisting of the solvents (c), (d), and (e).
[T5] The use according to any one of [T1] to [T4], wherein the topical agent for onychomycosis or the medication contains, as a solvent, at least one selected from the group consisting of the solvents (d) and (e).
[T6] The use according to any one of [T1] to [T5], wherein the dibasic acid diester is a diester of a dibasic acid and an alcohol having 1 to 4 carbon atoms.
[T7] The use according to any one of [T1] to [T6], wherein the topical agent for onychomycosis or the medication further contains polyvinylpyrrolidone.
[T8] The use according to any one of [T1] to [T7], wherein the topical agent for onychomycosis or the medication further contains an acid as a pH adjusting agent.
[T9] The use according to [T8], wherein the pH adjusting agent is lactic acid or citric acid.
[T10] The use according to any one of [T1] to [T9], wherein in the topical agent for onychomycosis or the medication, a content of the at least one selected from the group consisting of the compound (1) and salts thereof is 0.1 to 20% by mass, and a content of the at least one selected from the group consisting of the solvents (a) to (g) is 60 to 99.9% by mass.

### Examples

Examples and comparative examples will now be described for further specifically illustrating the topical agent of the present disclosure, and it is noted that the present disclosure is not limited to these examples.

As a compound (1) used in the following examples, a monohydrate crystal was used, which was prepared on the basis of the description of Production Example 2 of WO2023/106320.

### [Example 1]

In accordance with the formulation described below, components were mixed in a glass screw-cap tube (capacity: 10 mL to 30 mL) at room temperature (about 25°C), and the resultant mixture was manually shaken until the solid components dissolved, to prepare a topical agent of Example 1.

| | |
|---|---|
| Compound (1) | 5 parts by mass |
| Isopropyl alcohol | 47.5 parts by mass |
| Diisopropyl adipate | 47.5 parts by mass |

### [Evaluation Test: Suppression of Crystal Precipitation]

Each topical agent of Examples and Comparative Examples was tested on the evaluation items described below.

### [Examples 2 to 81, and Comparative Examples 1 to 6]

Topical agents of Examples 2 to 81 and Comparative Examples 1 to 6 were prepared in the same manner as in Example 1 except that the formulations were changed as shown in Tables 1 through 23 for respective evaluation tests described below.

### <Suppression of Crystal Precipitation in Formulation>

Each of the obtained topical agents was allowed to stand at room temperature (about 25°C) for 24 ± 1 hours, and thereafter, the presence or absence of crystal precipitation was visually inspected and evaluated based on the following criteria:
A: No crystal precipitation was observed after 24 ± 1 hours, with a clear appearance.
X: Crystal precipitation was observed in 24 ± 1 hours.

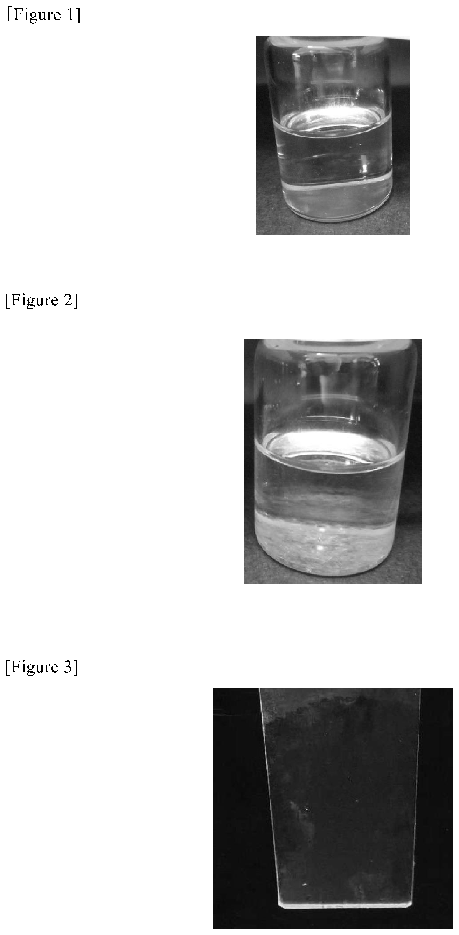

For reference, Figures 1 and 2 respectively show photographs of the state of the topical agents of Example 1 and Comparative Example 3 after 24 hours of standing, as evaluated for the test item "Suppression of Crystal Precipitation in Formulation".

Example 1 is an example rated as "A", which had solubility, and as illustrated in Figure 1, it was confirmed that no crystal precipitation was observed with a clear appearance.

Comparative Example 3 is an example rated as "X", and as illustrated in Figure 2, it was confirmed that small crystals precipitated were observed at the bottom.

### <Suppression of Crystal Precipitation after Application>

A single drop (about 1 µL to 10 µL) of the obtained topical agent was gently placed onto a glass plate using a pipette to form a medicated spot, and after a prescribed period, the presence or absence of crystal precipitation was visually inspected, and was determined based on the following criteria:
A: No crystal precipitation was observed in the medicated spot on the glass plate 24 ± 1 hours after the dropwise application.
X: Crystal precipitation was observed within 24 ± 1 hours after the dropwise application.

For reference, Figures 3 to 5 respectively show photographs of the state of the topical agents of Example 22 and Comparative Examples 1 and 2 after the prescribed time period following the application of the topical agent, as evaluated for the test item "Suppression of Crystal Precipitation after Application".

Example 22 is an example rated as "A", in which the applied droplet was spread out and, as illustrated in Figure 3, it was confirmed that no crystalline material was observed even after the passage of time.

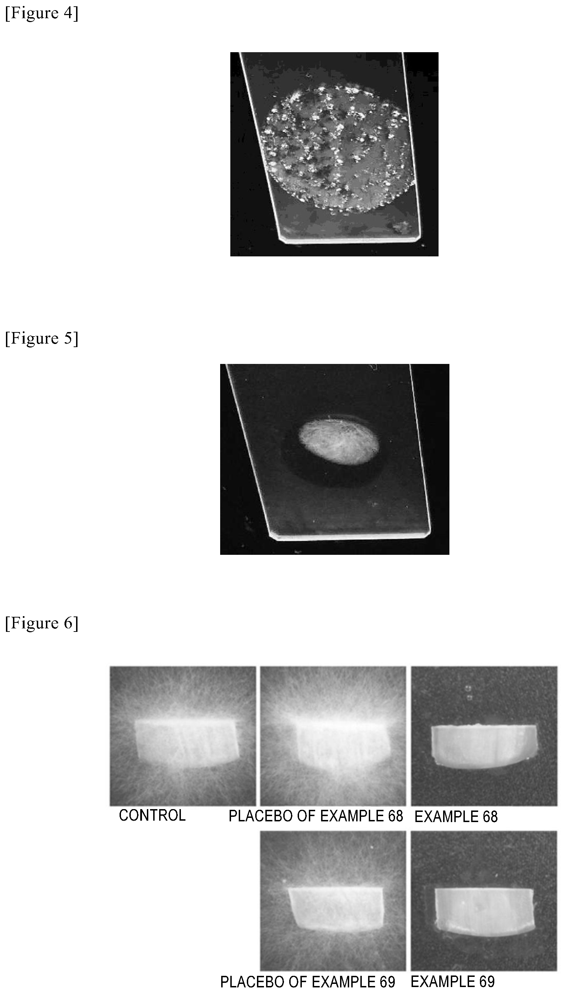

Comparative Examples 1 and 2 are examples rated as "X". As illustrated in Figure 4, precipitation of white crystalline material was observed along the contour of the medicated spot in Comparative Example 1, and as illustrated in Figure 5, precipitation of needle-like crystals was observed in Comparative Example 2.

### [Evaluation Test Results: Suppression of Crystal Precipitation]

Evaluation results of the suppression of crystal precipitation of the respective topical agents of Examples and Comparative Examples are shown in Tables 1 to 17, along with the respective formulations.

In each table, "IPA" stands for isopropyl alcohol, "EtOH" stands for ethanol, "DMSO" stands for dimethyl sulfoxide, "NMP" stands for N-methyl-2-pyrrolidone, "PVP" stands for polyvinylpyrrolidone, "BzOH" stands for benzyl alcohol, "PG" stands for propylene glycol, "PEG" stands for polyethylene glycol, and "TEG" stands for triethylene glycol. As the polyvinylpyrrolidone (PVP), Polyvinylpyrrolidone K 30 (weight average molecular weight: 40,000) manufactured by Tokyo Chemical Industry Co., Ltd. was used. As the polyethylene glycol (PEG), Polyethylene Glycol 300 (number average molecular weight: 300) manufactured by FUJIFILM Wako Pure Chemical Corporation was used. The same notations apply to Table 18 and subsequent tables described below.

**[Table 1]**

| | Example 1 | Example 2 | Comparative Example 1 | Comparative Example 2 | Comparative Example 3 |
|---|---|---|---|---|---|
| Compound (1) | 5.0 | 5.0 | 5.0 | 5.0 | 5.0 |
| IPA | 47.5 | - | 95.0 | - | 47.5 |
| Diisopropyl Adipate | 47.5 | 95.0 | - | - | - |
| NMP | - | - | - | 95.0 | 47.5 |
| Total | 100 | 100 | 100 | 100 | 100 |
| Suppression of crystal precipitation in formulation | A | A | A | A | X |
| Suppression of crystal precipitation after application | A | A | X | X | X |

**[Table 2]**

| | Example 3 | Comparative Example 4 | Comparative Example 5 |
|---|---|---|---|
| Compound (1) | 5.0 | 5.0 | 5.0 |
| EtOH | 47.5 | 95.0 | 47.5 |
| Diisopropyl Adipate | 47.5 | - | - |
| NMP | - | - | 47.5 |
| Total | 100 | 100 | 100 |
| Suppression of crystal precipitation in formulation | A | A | A |
| Suppression of crystal precipitation after application | A | X | X |

**[Table 3]**

| | Example 4 | Example 5 | Example 6 | Example 7 | Comparative Example 6 |
|---|---|---|---|---|---|
| Compound (1) | 5.0 | 5.0 | 5.0 | 5.0 | 5.0 |
| IPA | 47.5 | - | 71.25 | - | - |
| EtOH | - | 47.5 | - | - | - |
| DMSO | 47.5 | 47.5 | 23.75 | 47.5 | 95.0 |
| Diisopropyl Adipate | - | - | - | 47.5 | - |
| Total | 100 | 100 | 100 | 100 | 100 |
| Suppression of crystal precipitation in formulation | A | A | A | A | A |
| Suppression of crystal precipitation after application | A | A | A | A | X |

**[Table 4]**

| | Example 8 | Example 9 | Example 10 | Example 11 |
|---|---|---|---|---|
| Compound (1) | 5.0 | 5.0 | 5.0 | 5.0 |
| IPA | 57.0 | 31.730 | 19.0 | 19.0 |
| DMSO | 19.0 | 31.635 | 19.0 | 57.0 |
| Diisopropyl Adipate | 19.0 | 31.635 | 57.0 | 19.0 |
| Total | 100 | 100 | 100 | 100 |
| Suppression of crystal precipitation in formulation | A | A | A | A |
| Suppression of crystal precipitation after application | A | A | A | A |

**[Table 5]**

| | Example 12 | Example 13 | Example 14 | Example 15 |
|---|---|---|---|---|
| Compound (1) | 5.0 | 5.0 | 5.0 | 5.0 |
| EtOH | 57.0 | 31.730 | 19.0 | 19.0 |
| DMSO | 19.0 | 31.635 | 19.0 | 57.0 |
| Diisopropyl Adipate | 19.0 | 31.635 | 57.0 | 19.0 |
| Total | 100 | 100 | 100 | 100 |
| Suppression of crystal precipitation in formulation | A | A | A | A |
| Suppression of crystal precipitation after application | A | A | A | A |

**[Table 6]**

| | Example 16 | Example 17 | Example 18 | Example 19 |
|---|---|---|---|---|
| Compound (1) | 5.0 | 5.0 | 5.0 | 5.0 |
| IPA | 71.25 | 71.25 | 71.25 | 71.25 |
| Diisopropyl Adipate | 23.75 | - | - | - |
| Diisobutyl Adipate | - | 23.75 | - | - |
| Dibutyl Fumarate | - | - | 23.75 | - |
| Diethyl maleate | - | - | - | 23.75 |
| Total | 100 | 100 | 100 | 100 |
| Suppression of crystal precipitation in formulation | A | A | A | A |
| Suppression of crystal precipitation after application | A | A | A | A |

**[Table 7]**

| | Example 20 | Example 21 | Example 22 | Example 23 |
|---|---|---|---|---|
| Compound (1) | 5.0 | 5.0 | 5.0 | 5.0 |
| IPA | 47.5 | 47.5 | 47.5 | 47.5 |
| DMSO | 23.75 | 23.75 | 23.75 | 23.75 |
| Diisopropyl Adipate | 23.75 | - | - | - |
| Diisobutyl Adipate | - | 23.75 | - | - |
| Dibutyl Fumarate | - | - | 23.75 | - |
| Diethyl maleate | - | - | - | 23.75 |
| Total | 100 | 100 | 100 | 100 |
| Suppression of crystal precipitation in formulation | A | A | A | A |
| Suppression of crystal precipitation after application | A | A | A | A |

**[Table 8]**

| | Example 24 | Example 25 | Example 26 |
|---|---|---|---|
| Compound (1) | 5.0 | 5.0 | 5.0 |
| EtOH | 71.25 | 71.25 | 71.25 |
| Diisopropyl Adipate | 23.75 | - | - |
| Diisobutyl Adipate | - | 23.75 | - |
| Dibutyl Fumarate | - | - | 23.75 |
| Total | 100 | 100 | 100 |
| Suppression of crystal precipitation in formulation | A | A | A |
| Suppression of crystal precipitation after application | A | A | A |

**[Table 9]**

| | Example 27 | Example 28 | Example 29 |
|---|---|---|---|
| Compound (1) | 5.0 | 5.0 | 5.0 |
| EtOH | 47.5 | 47.5 | 47.5 |
| DMSO | 23.75 | 23.75 | 23.75 |
| Diisopropyl Adipate | 23.75 | - | - |
| Diisobutyl Adipate | - | 23.75 | - |
| Dibutyl Fumarate | - | - | 23.75 |
| Total | 100 | 100 | 100 |
| Suppression of crystal precipitation in formulation | A | A | A |
| Suppression of crystal precipitation after application | A | A | A |

**[Table 10]**

| | Example 30 | Example 31 | Example 32 | Example 33 |
|---|---|---|---|---|
| Compound (1) | 10.00 | 10.00 | 5.00 | 5.00 |
| EtOH | 48.52 | - | - | - |
| IPA | - | 48.52 | 53.90 | 44.50 |
| DMSO | 7.54 | 7.54 | 8.40 | 23.00 |
| Diisopropyl Adipate | 25.44 | 25.44 | 28.20 | 23.00 |
| PVP | 0.50 | 0.50 | 0.50 | 0.50 |
| BzOH | 4.00 | 4.00 | - | - |
| Lactic Acid | 4.00 | 4.00 | 4.00 | 4.00 |
| Total | 100 | 100 | 100 | 100 |
| Suppression of crystal precipitation in formulation | A | A | A | A |
| Suppression of crystal precipitation after application | A | A | A | A |

**[Table 11]**

| | Example 34 | Example 35 | Example 36 | Example 37 |
|---|---|---|---|---|
| Compound (1) | 5.00 | 5.00 | 5.00 | 5.00 |
| EtOH | - | 53.90 | 44.50 | 45.50 |
| IPA | 45.50 | - | - | - |
| DMSO | - | 8.40 | 23.00 | - |
| Diisopropyl Adipate | 45.00 | 28.20 | 23.00 | 45.00 |
| PVP | 0.50 | 0.50 | 0.50 | 0.50 |
| Lactic Acid | 4.00 | 4.00 | 4.00 | 4.00 |
| Total | 100 | 100 | 100 | 100 |
| Suppression of crystal precipitation in formulation | A | A | A | A |
| Suppression of crystal precipitation after application | A | A | A | A |

**[Table 12]**

| | Example 38 | Example 39 | Example 40 |
|---|---|---|---|
| Compound (1) | 5.0 | 5.0 | 5.0 |
| EtOH | 47.5 | 47.5 | 47.5 |
| PG | 47.5 | - | - |
| PEG | - | 47.5 | - |
| TEG | - | - | 47.5 |
| Total | 100 | 100 | 100 |
| Suppression of crystal precipitation in formulation | A | A | A |
| Suppression of crystal precipitation after application | A | A | A |

**[Table 13]**

| | Example 41 | Example 42 | Example 43 | Example 44 | Example 45 |
|---|---|---|---|---|---|
| Compound (1) | 5.0 | 5.0 | 5.0 | 5.0 | 5.0 |
| IPA | 66.5 | 43.5 | 43.5 | 64.5 | 64.5 |
| PG | 8.0 | - | 43.0 | - | 22.0 |
| Diisopropyl Adipate | 12.0 | 43.0 | - | 22.0 | - |
| PVP | 0.5 | 0.5 | 0.5 | 0.5 | 0.5 |
| BzOH | 4.0 | 4.0 | 4.0 | 4.0 | 4.0 |
| Lactic Acid | 4.0 | 4.0 | 4.0 | 4.0 | 4.0 |
| Total | 100 | 100 | 100 | 100 | 100 |
| Suppression of crystal precipitation in formulation | A | A | A | A | A |
| Suppression of crystal precipitation after application | A | A | A | A | A |

**[Table 14]**

| | Example 46 | Example 47 | Example 48 | Example 49 | Example 50 |
|---|---|---|---|---|---|
| Compound (1) | 5.0 | 5.0 | 5.0 | 5.0 | 5.0 |
| IPA | 42.5 | 58.5 | 77.5 | 51.5 | 51.5 |
| PG | 22.0 | 14.0 | 4.5 | 27.0 | 8.0 |
| Diisopropyl Adipate | 22.0 | 14.0 | 4.5 | 8.0 | 27.0 |
| PVP | 0.5 | 0.5 | 0.5 | 0.5 | 0.5 |
| BzOH | 4.0 | 4.0 | 4.0 | 4.0 | 4.0 |
| Lactic Acid | 4.0 | 4.0 | 4.0 | 4.0 | 4.0 |
| Total | 100 | 100 | 100 | 100 | 100 |
| Suppression of crystal precipitation in formulation | A | A | A | A | A |
| Suppression of crystal precipitation after application | A | A | A | A | A |

**[Table 15]**

| | Example 51 | Example 52 | Example 53 | Example 54 | Example 55 |
|---|---|---|---|---|---|
| Compound (1) | 5.0 | 5.0 | 5.0 | 5.0 | 5.0 |
| EtOH | 66.5 | 43.5 | 43.5 | 64.5 | 64.5 |
| PG | 8.0 | - | 43.0 | - | 22.0 |
| Diisopropyl Adipate | 12.0 | 43.0 | - | 22.0 | - |
| PVP | 0.5 | 0.5 | 0.5 | 0.5 | 0.5 |
| BzOH | 4.0 | 4.0 | 4.0 | 4.0 | 4.0 |
| Lactic Acid | 4.0 | 4.0 | 4.0 | 4.0 | 4.0 |
| Total | 100 | 100 | 100 | 100 | 100 |
| Suppression of crystal precipitation in formulation | A | A | A | A | A |
| Suppression of crystal precipitation after application | A | A | A | A | A |

**[Table 16]**

| | Example 56 | Example 57 | Example 58 | Example 59 | Example 60 |
|---|---|---|---|---|---|
| Compound (1) | 5.0 | 5.0 | 5.0 | 5.0 | 5.0 |
| EtOH | 42.5 | 58.5 | 77.5 | 51.5 | 51.5 |
| PG | 22.0 | 14.0 | 4.5 | 27.0 | 8.0 |
| Diisopropyl Adipate | 22.0 | 14.0 | 4.5 | 8.0 | 27.0 |
| PVP | 0.5 | 0.5 | 0.5 | 0.5 | 0.5 |
| BzOH | 4.0 | 4.0 | 4.0 | 4.0 | 4.0 |
| Lactic Acid | 4.0 | 4.0 | 4.0 | 4.0 | 4.0 |
| Total | 100 | 100 | 100 | 100 | 100 |
| Suppression of crystal precipitation in formulation | A | A | A | A | A |
| Suppression of crystal precipitation after application | A | A | A | A | A |

**[Table 17]**

| | Example 61 | Example 62 | Example 63 | Example 64 | Example 65 | Example 66 |
|---|---|---|---|---|---|---|
| Compound (1) | 5.0 | 5.0 | 5.0 | 5.0 | 5.0 | 5.0 |
| IPA | 66.5 | 43.5 | 51.5 | - | - | - |
| EtOH | - | - | - | 66.5 | 43.5 | 51.5 |
| PEG | 8.0 | 43.0 | 27.0 | 8.0 | 43.0 | 27.0 |
| Diisopropyl Adipate | 12.0 | - | 8.0 | 12.0 | - | 8.0 |
| PVP | 0.5 | 0.5 | 0.5 | 0.5 | 0.5 | 0.5 |
| BzOH | 4.0 | 4.0 | 4.0 | 4.0 | 4.0 | 4.0 |
| Lactic Acid | 4.0 | 4.0 | 4.0 | 4.0 | 4.0 | 4.0 |
| Total | 100 | 100 | 100 | 100 | 100 | 100 |
| Suppression of crystal precipitation in formulation | A | A | A | A | A | A |
| Suppression of crystal precipitation after application | A | A | A | A | A | A |

The results shown in Table 1 will be discussed.

The formulation of Example 1 contained isopropyl alcohol and diisopropyl adipate as the solvent (this solvent is encompassed in the concept of the "solvent (b)"). The solubility of the active ingredient in the formulation was good, with no crystal precipitation observed, and no crystal precipitation occurred also after the application.

The formulation of Example 2 contained only diisopropyl adipate as the solvent (this solvent is encompassed in the concept of the "solvent (a)"). Similarly to Example 1, it was confirmed that the effects of the present disclosure were exhibited. Accordingly, it was found that diisopropyl adipate produces the effects of the present disclosure even when used alone as the solvent.

The formulation of Comparative Example 1 contained only isopropyl alcohol as the solvent, and the formulation of Comparative Example 2 contained only N-methyl-2-pyrrolidone as the solvent. Although these formulations had good solubility of the active ingredient in the formulation with no crystal precipitation observed, crystal precipitation occurred after the application. Accordingly, it is understood that the suppression of crystal precipitation after the application is not solely attributable to the good solubility provided by the solvent.

The formulation of Comparative Example 3 contained isopropyl alcohol and N-methyl-2-pyrrolidone as the solvent. The solubility of the active ingredient in the formulation was insufficient, crystal precipitation was observed, and crystal precipitation also occurred after the application. Since a combination of N-methyl-2-pyrrolidone with another solvent led to crystal precipitation in the formulation, it was suggested that it is not preferable, in the present disclosure, to incorporate N-methyl-2-pyrrolidone as a solvent.

The results shown in Table 2 will be discussed.

The formulation of Example 3 contained ethanol and diisopropyl adipate as the solvent (this solvent is encompassed in the concept of the "solvent (b)"), and similarly to Example 1, in which isopropyl alcohol was used, it was confirmed that the effects of the present disclosure were exhibited.

The formulation of Comparative Example 4 contained only ethanol as the solvent. Although the formulation had good solubility of the active ingredient with no crystal precipitation observed, crystal precipitation occurred after the application. This result suggests, similarly to the results shown in Table 1, that the suppression of crystal precipitation after the application is not solely attributable to the good solubility provided by the solvent.

The formulation of Comparative Example 5 contained ethanol and N-methyl-2-pyrrolidone as the solvent. The solubility of the active ingredient in the formulation was insufficient, crystal precipitation was observed, and crystal precipitation also occurred after the application. This result also exhibits a trend similar to that shown in Table 1, and suggests that it is not preferable, in the present disclosure, to incorporate N-methyl-2-pyrrolidone as a solvent.

The results shown in Table 3 will be discussed.

The formulation of Examples 4 and 6 contained isopropyl alcohol and dimethyl sulfoxide as the solvent (this solvent is encompassed in the concept of the "solvent (c)"), the formulation of Example 5 contained ethanol and dimethyl sulfoxide as the solvent (this solvent is encompassed in the concept of the "solvent (c)"), and the formulation of Example 7 contained dimethyl sulfoxide and diisopropyl adipate as the solvent (this solvent is encompassed in the concept of the "solvent (d)"). It was confirmed that the effects of the present disclosure were exhibited also by using any combination of these various solvents.

The results shown in Tables 4 and 5 will be discussed.

The formulation of Examples 8 to 11 contained isopropyl alcohol, dimethyl sulfoxide, and diisopropyl adipate as the solvent (this solvent is encompassed in the concept of the "solvent (e)"), and it was confirmed that the effects of the present disclosure were exhibited at various blending ratios.

The formulation of Examples 12 to 15 contained ethanol, dimethyl sulfoxide, and diisopropyl adipate as the solvent (this solvent is encompassed in the concept of the "solvent (e)"), and it was confirmed, also in these Examples, that the effects of the present disclosure were exhibited at various blending ratios.

The results shown in Tables 6 to 9 will be discussed.

Examples 16 to 29 were based on formulations containing diisopropyl adipate to examine formulations in which the diisopropyl adipate was replaced with various dibasic acid diesters (these solvents are encompassed in the concept of the "solvent (b)" or the "solvent (e)"). It was confirmed that the effects of the present disclosure were exhibited also by various dibasic acid diesters other than diisopropyl adipate similarly to diisopropyl adipate.

The results shown in Tables 10 and 11 will be discussed.

The formulation of Examples 30 to 33 contained isopropyl alcohol or ethanol, dimethyl sulfoxide, and diisopropyl adipate as the solvent (this solvent is encompassed in the concept of the "solvent (e)"), and the formulation of Examples 34 to 37 contained isopropyl alcohol or ethanol, and diisopropyl adipate as the solvent (this solvent is encompassed in the concept of the "solvent (b)"). In these Examples, polyvinylpyrrolidone, benzyl alcohol, lactic acid and others were incorporated as suitable optional ingredients. The solubility of the active ingredient in the formulation was good, with no crystal precipitation observed, and no crystal precipitation occurred also after the application. These results demonstrate that the aforementioned optional ingredients may contribute to further improvement of the physical properties as a topical agent for onychomycosis, without impairing the effects of the present disclosure.

The results shown in Table 12 will be discussed.

The formulation of Examples 38 to 40 contained ethanol and various glycols as the solvent (this solvent is encompassed in the concept of the "solvent (f)"), and it was confirmed that the effects of the present disclosure were exhibited in combinations of a lower alcohol with various glycols.

The results shown in Tables 13 to 17 will be discussed.

It was confirmed in Examples 41 to 66 that the effects of the present disclosure were exhibited by various combinations encompassed by the concepts of the solvent (b), the solvent (f), or the solvent (g). In particular, it was confirmed that the effects of the present disclosure were not inhibited even when incorporating various optional ingredients, and also that the effects of the present disclosure were exhibited at various blending ratios.

### [Evaluation Test: Subungual Efficacy Test]

### <Preparation of Conidial Suspension>

The fungal bodies of *Trichophyton rubrum* (CBS 118892), cultured on Sabouraud dextrose agar medium, were scraped off and transferred into a tube, and 500 µL of physiological saline was added thereto to disperse the fungal bodies. The resultant was filtered through a cell strainer (mesh size: 40 µm), and the resultant filtrate was centrifuged (13,000 rpm for 5 minutes) to collect a precipitate. The collected precipitate was washed with physiological saline, and then dispersed in physiological saline to prepare a fungal body suspension.

The fungal body suspension was spread onto a rye medium, and cultured at 28°C. After the culture, 5 mL of physiological saline was added to the resulting bacterial flora, and the surface was scraped with a scraper to collect a fungal suspension. After stirring the fungal suspension by vortexing, the resultant was filtered through a 100 µm filter to obtain a conidial suspension. The number of conidia therein was examined under a microscope, and the suspension was used for testing.

### <Preparation of Human Nail>

A nail was disinfected by washing with 70% ethanol, then immersed in 100% ethanol for 1 hour to remove moisture, and subsequently, was air-dried for 1 hour under germicidal lamp irradiation in a biosafety cabinet.

### <Treatment of Human Nail with Drug Solution>

The human nail was placed individually in a dish (35 mm in diameter), and 0.5 µL of a drug solution was applied from the upper plate side of the nail plate in such a manner that the solution stayed only on the upper plate side and did not run down the sides. After the surface was dry, the nail was kept in an incubator at 25°C to allow the drug solution to penetrate into the human nail.

The above operation was performed once a day, and the drug solution was applied 1 to 7 times in total.

As the drug solution, the topical agents for onychomycosis of Examples 3, 5, 7, 27, 38 to 40, 50, 51, 53, 59, 60, and 67 to 71 were used.

### <Preparation of Culture Medium for Test>

Ampicillin was added to physiological saline containing 1.5% agarose to prepare a medium for a subungual efficacy test.

### <Subungual Efficacy Test>

The conidial suspension (1 × 10⁵/1 µL) was spot-inoculated onto the medium for the test. The human nail, which had been treated with the drug solution, was then placed thereon, such that the bottom plate side of the nail plate (the opposite side from the upper plate side of the nail plate, to which the drug solution had been applied) was in contact with the conidia and the medium, and the resultant was then cultured in an incubator at 28°C.

After 7 to 10 days of culture, the growth of trichophyton was observed, and determination was made based on the following criteria:
A: No hyphal growth was observed.
X: Hyphal growth was observed.

For reference, Figure 6 shows photographs of the state around the nail in the test medium after seven applications (once a day) of the drug solution in Examples 68 and 69, their corresponding placebos, and a control, in which no drug solution was applied to the nail, in "Subungual Efficacy Test".

It was confirmed that no hyphal growth was observed in Examples 68 and 69 shown in Figure 6, which was accordingly rated as "A". On the other hand, hyphal growth was observed in the placebos and the control, which was accordingly rate as "X". In this manner, the rating of "A" and "X" can be conducted with relative ease and objectivity by visual observation.

### [Results of Evaluation Test: Subungual Efficacy Test]

Tables 18 to 21 show the evaluation test results of the subungual efficacy test on the human nail of the topical agents for onychomycosis of respective Examples used as the test drug solutions, along with their formulations.

The results of Examples 3, 5, 7, 27, 38 to 40, 50, and 60 are based on three treatments with the drug solution, the result of Example 67 is based on seven treatments with the drug solution, and all other results are based on a single treatment with the drug solution.

**[Table 18]**

| | Example 3 | Example 5 | Example 7 | Example 27 | Example 67 |
|---|---|---|---|---|---|
| Compound (1) | 5.0 | 5.0 | 5.0 | 5.0 | 10.0 |
| EtOH | 47.5 | 47.5 | - | 47.5 | - |
| DMSO | - | 47.5 | 47.5 | 23.75 | - |
| Diisopropyl Adipate | 47.5 | - | 47.5 | 23.75 | 90.0 |
| Total | 100 | 100 | 100 | 100 | 100 |
| Subungual efficacy | A | A | A | A | A |

**[Table 19]**

| | Example 38 | Example 39 | Example 40 |
|---|---|---|---|
| Compound (1) | 5.0 | 5.0 | 5.0 |
| EtOH | 47.5 | 47.5 | 47.5 |
| PG | 47.5 | - | - |
| PEG | - | 47.5 | - |
| TEG | - | - | 47.5 |
| Total | 100 | 100 | 100 |
| Subungual efficacy | A | A | A |

**[Table 20]**

| | Example 50 | Example 51 | Example 53 | Example 59 | Example 60 |
|---|---|---|---|---|---|
| Compound (1) | 5.0 | 5.0 | 5.0 | 5.0 | 5.0 |
| IPA | 51.5 | - | - | - | - |
| EtOH | - | 66.5 | 43.5 | 51.5 | 51.5 |
| PG | 8.0 | 8.0 | 43.0 | 27.0 | 8.0 |
| Diisopropyl Adipate | 27.0 | 12.0 | - | 8.0 | 27.0 |
| PVP | 0.5 | 0.5 | 0.5 | 0.5 | 0.5 |
| BzOH | 4.0 | 4.0 | 4.0 | 4.0 | 4.0 |
| Lactic Acid | 4.0 | 4.0 | 4.0 | 4.0 | 4.0 |
| Total | 100 | 100 | 100 | 100 | 100 |
| Subungual efficacy | A | A | A | A | A |

**[Table 21]**

| | Example 68 | Example 69 | Example 70 | Example 71 |
|---|---|---|---|---|
| Compound (1) | 5.0 | 5.0 | 5.0 | 5.0 |
| IPA | 51.5 | - | - | - |
| EtOH | - | 51.5 | 70.0 | 70.0 |
| DMSO | 8.0 | 8.0 | - | - |
| PG | - | - | 8.0 | 8.0 |
| Diisopropyl Adipate | 27.0 | 27.0 | 12.0 | 12.0 |
| PVP | 0.5 | 0.5 | 0.5 | 0.5 |
| BzOH | 4.0 | 4.0 | 4.0 | 4.0 |
| Lactic Acid | 4.0 | 4.0 | 0.5 | - |
| Citric acid monohydrate | - | - | - | 0.5 |
| Total | 100 | 100 | 100 | 100 |
| Subungual efficacy | A | A | A | A |

### [Discussion: Subungual Efficacy Test]

As understood from the results of Examples, the subungual efficacy was confirmed for the topical agent for onychomycosis containing the compound (1) with the specific solvent of the present disclosure. Since the operation of the treatment with the drug solution was conducted so that the drug solution stayed only on the upper surface of the nail, the above-described test results indicate that the topical agent for onychomycosis of the present disclosure not only exhibits the effect of inhibiting hyphal growth under the nail but also achieves nail penetration.

### [Evaluation Test: Fungicidal Activity Test on Human Nail]

This test was performed in the same manner as in "Evaluation Test: Subungual Efficacy Test" described above except for the following.

### <Treatment of Human Nail with Drug Solution>

A human nail was placed individually in a dish (35 mm in diameter), and 0.5 µL of a drug solution was applied from the upper plate side of the nail plate in the same manner as in the above-described test. After the surface was dry, the nail was kept in an incubator at 25°C to allow the drug solution to penetrate into the human nail.

The above operation was performed only once, and the penetration time was 7 days after the application.

As the drug solution, the topical agent for onychomycosis of Example 68 was used.

### <Culture Test>

A cellophane sheet (10 mm in length × 10 mm in width × 50 µm in thickness) was placed on a medium for test, and a conidial suspension (1 × 10⁵ /1 µL) was spot-inoculated thereon. The human nail, into which the drug solution had been penetrated, was then placed thereon, such that the bottom plate side of the nail plate was in contact with the conidia and the cellophane sheet, and the resultant was then cultured at 28°C for 24 hours. Thereafter, the conidia were stained with a 4',6-diamidino-2-phenylindole dihydrochloride (DAPI) solution to determine viability.

### [Results of Evaluation Test: Fungicidal Activity Test on Human Nail]

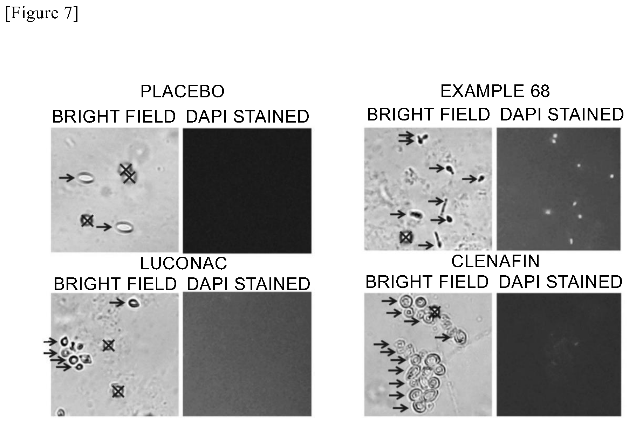

The result of Example 68 is illustrated in Figure 7. For comparison, the results of commercially available nail topical solutions, "LUCONAC" (R) 5% topical solution, manufactured by Sato Pharmaceutical Co., Ltd., and "CLENAFIN" (R) 10% topical solution, manufactured by Kaken Pharmaceutical Co., Ltd., and the result of a placebo corresponding to Example 68 are also illustrated in Figure 7.

### [Discussion: Fungicidal Activity Test on Human Nail]

DAPI does not permeate through the cell membrane of living cells, but permeates through the membrane of dead cells because the membrane is altered. This principle was utilized in this test to determine the viability of the subungual conidia.

For the topical agent for onychomycosis of Example 68, it was observed that the subungual conidia were stained by DAPI in the dark field. In contrast, no staining of the conidia was observed for the placebo and the two commercially available drugs used for comparison.

Therefore, it was confirmed that the topical agent for onychomycosis of Example 68 successfully killed the subungual conidia, while the conidia remained viable when using the placebo and the two commercially available drugs.

Also in observation in the bright field, a certain degree of growth of the conidia, such as swelling, was observed for the placebo and the two commercially available drugs, but no growth of the conidia was observed for the topical agent for onychomycosis of Example 68.

Thus, while the conventional topical agents for onychomycosis possessed antifungal activity but not fungicidal activity, the topical agent for onychomycosis of the present disclosure possessed the fungicidal activity in addition to the antifungal activity, and in this respect, exhibited a remarkable effect not achieved by the conventional technologies.

### [Reference Test: Effect of Amount and Type of Acid Added as Optional Ingredient on Storage Stability]

### <Effect of Difference in Amount of Lactic Acid Added on Storage Stability>

The effect of difference in the amount of lactic acid added on the storage stability was investigated using topical agents of Examples 72 to 76 (referred to as the Example 49-based group), in which the amount of lactic acid added was varied from that in Example 49 (including a case where no lactic acid was added), and topical agents of Examples 77 to 81 (referred to as the Example 59-based group), in which the amount of lactic acid added was varied from that in Example 59 (including a case where no lactic acid was added). The respective formulations are shown in Tables 22 and 23 below.

**[Table 22]**

| | Example 72 | Example 73 | Example 74 | Example 75 | Example 49 | Example 76 |
|---|---|---|---|---|---|---|
| Compound (1) | 5.0 | 5.0 | 5.0 | 5.0 | 5.0 | 5.0 |
| IPA | 55.5 | 55.0 | 54.5 | 53.5 | 51.5 | 47.5 |
| PG | 27.0 | 27.0 | 27.0 | 27.0 | 27.0 | 27.0 |
| Diisopropyl Adipate | 8.0 | 8.0 | 8.0 | 8.0 | 8.0 | 8.0 |
| PVP | 0.5 | 0.5 | 0.5 | 0.5 | 0.5 | 0.5 |
| BzOH | 4.0 | 4.0 | 4.0 | 4.0 | 4.0 | 4.0 |
| Lactic Acid | | 0.5 | 1.0 | 2.0 | 4.0 | 8.0 |
| Total | 100 | 100 | 100 | 100 | 100 | 100 |

**[Table 23]**

| | Example 77 | Example 78 | Example 79 | Example 80 | Example 59 | Example 81 |
|---|---|---|---|---|---|---|
| Compound (1) | 5.0 | 5.0 | 5.0 | 5.0 | 5.0 | 5.0 |
| EtOH | 55.5 | 55.0 | 54.5 | 53.5 | 51.5 | 47.5 |
| PG | 27.0 | 27.0 | 27.0 | 27.0 | 27.0 | 27.0 |
| Diisopropyl Adipate | 8.0 | 8.0 | 8.0 | 8.0 | 8.0 | 8.0 |
| PVP | 0.5 | 0.5 | 0.5 | 0.5 | 0.5 | 0.5 |
| BzOH | 4.0 | 4.0 | 4.0 | 4.0 | 4.0 | 4.0 |
| Lactic Acid | - | 0.5 | 1.0 | 2.0 | 4.0 | 8.0 |
| Total | 100 | 100 | 100 | 100 | 100 | 100 |

Next, a prepared sample solution (about 6 mL) was placed into a glass screw-cap tube (capacity: 10 mL), and the cap and body were sealed using a VALQUA tape and a vinyl tape. The resultant was stored for 4 weeks under conditions of 60°C and 80% RH. The sample solution after the storage was diluted with acetonitrile and analyzed by liquid chromatography. An area percentage was calculated based on a sum of peak areas obtained on the resultant chromatogram and a peak area of a hydrolysate, that is, a major decomposition product of the compound (1).

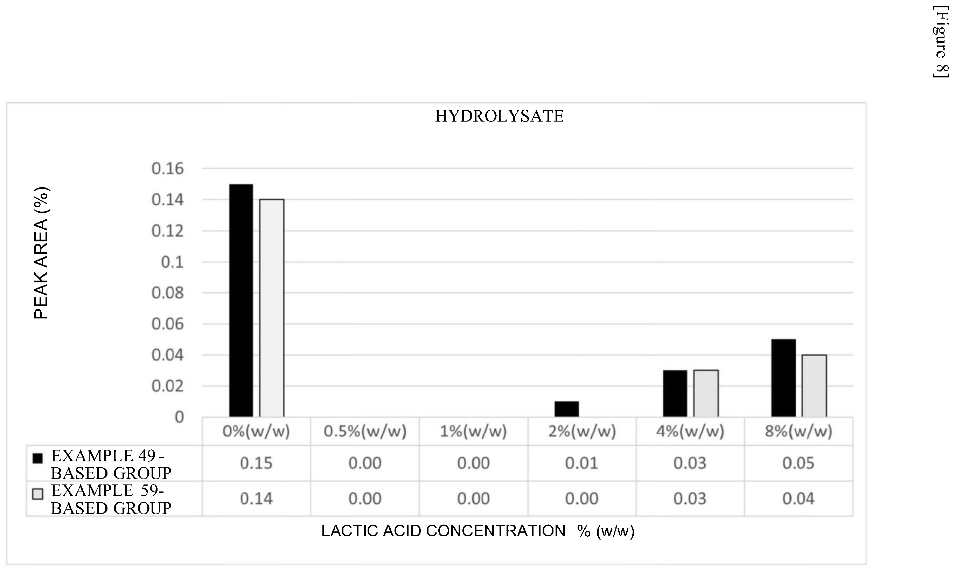

The results are illustrated in Figure 8.

### <Effect of Difference in Type of Acid on Storage Stability>

Sample solutions of Reference Examples 1 to 11 were prepared in the same manner as in Example 1, following formulations shown in Tables 24 to 26, which included the compound (1), a lower alcohol and an acid.

**[Table 24]**

| | Reference Example 1 | Reference Example 2 | Reference Example 3 | Reference Example 4 | Reference Example 5 | Reference Example 6 |
|---|---|---|---|---|---|---|
| Compound (1) | 5.0 | 5.0 | 5.0 | 5.0 | 5.0 | 5.0 |
| IPA | 94.5 | 94.0 | 93.0 | 94.95 | 94.9 | 94.8 |
| Lactic Acid | 0.5 | 1.0 | 2.0 | - | - | - |
| Phosphoric acid | - | - | - | 0.05 | 0.1 | 0.2 |
| Total | 100 | 100 | 100 | 100 | 100 | 100 |

**[Table 25]**

| | Reference Example 7 | Reference Example 8 | Reference Example 9 |
|---|---|---|---|
| Compound (1) | 5.0 | 5.0 | 5.0 |
| IPA | 94.9 | 94.8 | 94.6 |
| Hydrochloric acid (2M) | 0.1 | 0.2 | 0.4 |
| Total | 100 | 100 | 100 |

**[Table 26]**

| | Reference Example 10 | Reference Example 11 |
|---|---|---|
| Compound (1) | 5.0 | 5.0 |
| Ethanol | 94.8 | 94.96 |
| Citric acid | 0.2 | 0.04 |
| Total | 100 | 100 |

Next, in the same manner as in the test of "Effect of Difference in Amount of Lactic Acid Added on Storage Stability" described above, the analysis was performed by liquid chromatography, and an area percentage was calculated based on a sum of peak areas obtained on the resultant chromatogram and a peak area of a hydrolysate, that is, a major decomposition product of the compound (1).

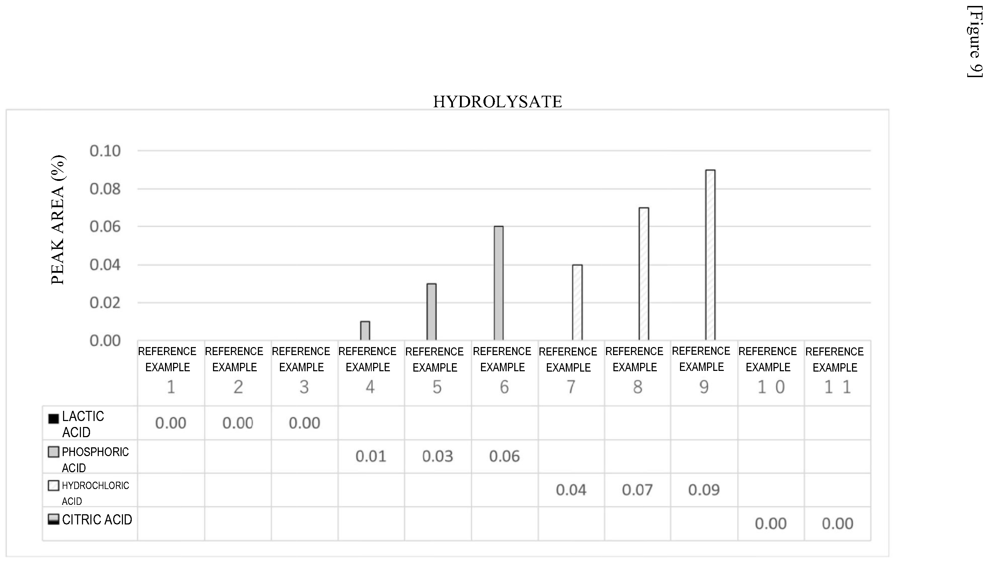

The results are illustrated in Figure 9.

### <Discussion>

It was found, from the results of the reference test described above, that the addition of an acid was advantageous for improving the storage stability of the compound (1), and that the addition of lactic acid or citric acid was particularly preferable. Regarding the amount added, a greater amount is not necessarily better, and for example, in the case of adding lactic acid, the mass percentage relative to the total amount of the topical agent is preferably 8% by mass or less, more preferably 4% by mass or less, and particularly preferably 0.5 to 2% by mass.

### Industrial Applicability

The present disclosure can be favorably used in the field of treatment or prevention of exacerbation of onychomycosis.

## Claims

1. A topical agent for onychomycosis comprising, as an active ingredient, at least one selected from the group consisting of a compound represented by the following formula (1) and salts thereof, and further comprising, as a solvent, at least one selected from the group consisting of the following solvents (a) to (g): 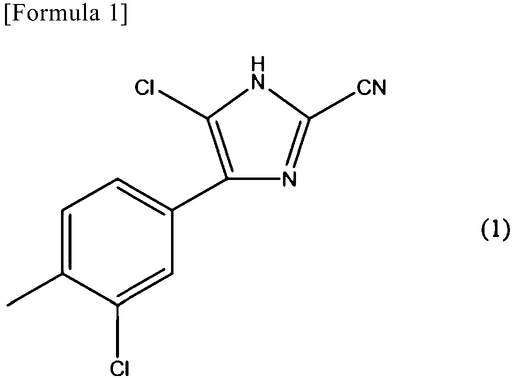 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol, and the solvent (g) is a lower alcohol, a glycol, and a dibasic acid diester.

2. The topical agent for onychomycosis according to claim 1, comprising, as a solvent, at least one selected from the group consisting of the solvents (a), (b), (d), and (e).

3. The topical agent for onychomycosis according to claim 1, comprising, as a solvent, at least one selected from the group consisting of the solvents (c), (d), and (e).

4. The topical agent for onychomycosis according to claim 1, comprising, as a solvent, at least one selected from the group consisting of the solvents (d) and (e).

5. The topical agent for onychomycosis according to claim 1, wherein the dibasic acid diester is a diester of a dibasic acid and an alcohol having 1 to 4 carbon atoms.

6. The topical agent for onychomycosis according to claim 1, further comprising polyvinylpyrrolidone.

7. The topical agent for onychomycosis according to claim 1, further comprising an acid as a pH adjusting agent.

8. The topical agent for onychomycosis according to claim 7, wherein the pH adjusting agent is lactic acid or citric acid.

9. The topical agent for onychomycosis according to claim 1, wherein a content of the at least one selected from the group consisting of the compound (1) and salts thereof is 0.1 to 20% by mass, and a content of the at least one selected from the group consisting of the solvents (a) to (g) is 60 to 99.9% by mass.

10. Use, for producing a topical agent for onychomycosis or a medication for treating or preventing exacerbation of onychomycosis, of a combination of at least one compound selected from the group consisting of a compound represented by the following formula (1) and salts thereof, and at least one solvent selected from the group consisting of the following solvents (a) to (g): 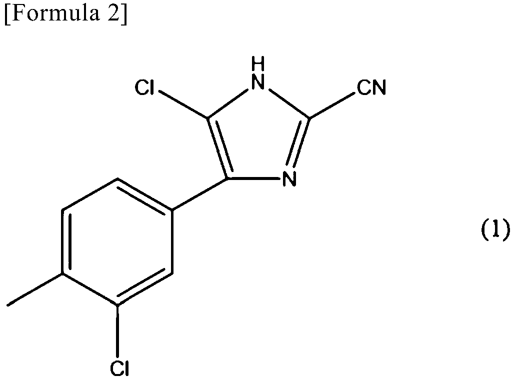 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.

11. Use, for producing a topical agent for onychomycosis or a medication for treating or preventing exacerbation of onychomycosis, of a composition comprising, as an active ingredient, at least one selected from the group consisting of a compound represented by the following formula (1) and salts thereof, and further comprising, as a solvent, at least one selected from the group consisting of the following solvents (a) to (g): 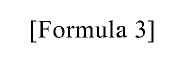 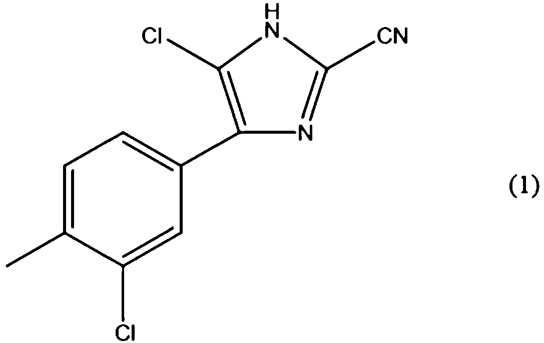 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.

12. A composition, for use in treatment or prevention of exacerbation of onychomycosis, comprising, as an active ingredient, at least one selected from the group consisting of a compound represented by the following formula (1) and salts thereof, and further comprising, as a solvent, at least one selected from the group consisting of the following solvents (a) to (g): 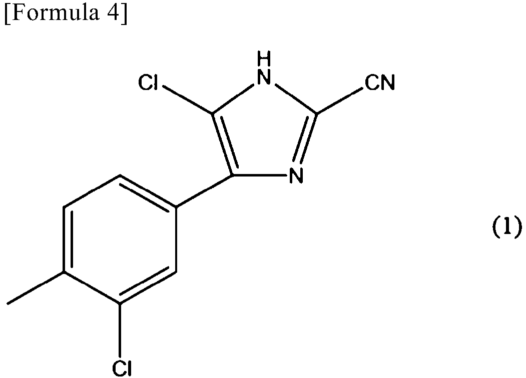 wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.

13. A method for treating onychomycosis in a subject, comprising locally applying an effective amount of a topical agent for onychomycosis to an affected nail of the subject,
wherein the topical agent for onychomycosis comprises, as an active ingredient, at least one selected from the group consisting of a compound represented by the following formula (1) and salts thereof, and further comprises, as a solvent, at least one selected from the group consisting of the following solvents (a) to (g): 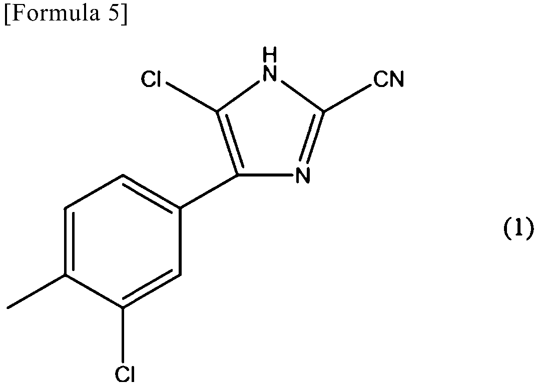
wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.

14. A method for preventing exacerbation of onychomycosis in a subject, comprising locally applying an effective amount of a topical agent for onychomycosis to an affected nail of the subject,
wherein the topical agent for onychomycosis comprises, as an active ingredient, at least one selected from the group consisting of a compound represented by the following formula (1) and salts thereof, and comprises, as a solvent, at least one selected from the group consisting of the following solvents (a) to (g): 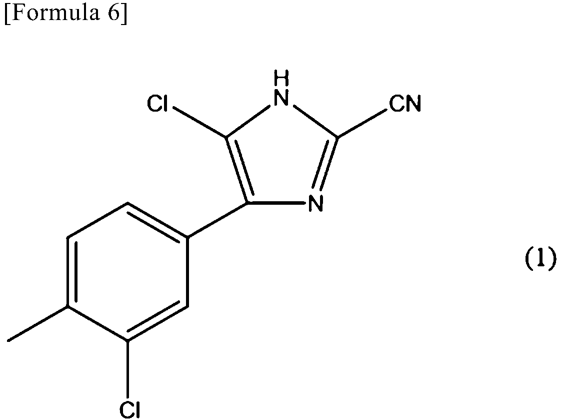
wherein the solvent (a) is a dibasic acid diester; the solvent (b) is a lower alcohol and a dibasic acid diester; the solvent (c) is a lower alcohol and dimethyl sulfoxide; the solvent (d) is dimethyl sulfoxide and a dibasic acid diester; the solvent (e) is a lower alcohol, dimethyl sulfoxide, and a dibasic acid diester; the solvent (f) is a lower alcohol, and a glycol; and the solvent (g) is a lower alcohol, a glycol, and a dibasic acid diester.
